# EUROPEAN PATENT APPLICATION

(11) **EP 3 141 211 A1**
(43) Date of publication of application: **15.03.2017**
(21) Application number: 14891195.1
(22) Date of filing: 08.05.2014
(51) Int. Cl.: A61C 17/20, A46B 15/00, A61C 3/03

(54) **ULTRASOUND SCALER CHIP, ULTRASOUND SCALER, AND TOOTH BRUSH**

(71) Applicant: Kanno, Minoru, Sendai-shi, Miyagi 980-0871 (JP)
(72) Inventor: KANNO, Taro, Sendai-shi Miyagi 980-8577 (JP)
(74) Representative: Plougmann Vingtoft a/s
(86) International application number: PCT/JP2014/062314
(87) International publication number: WO 2015/170381

(57) **Abstract**

An ultrasound scaler tip and an ultrasound scaler having a high sterilization effect are provided. A toothbrush having a reduced effect on the interior of the mouth is provided.

An ultrasound scaler tip 1 has a tip body 11 and an optical fiber 12. The tip body 11 has a tubular shape with a pointed-end opening and a rear-end opening, a rear end that can be attached to the ultrasound scaler body 2, and an intermediate curved part 16. The optical fiber 12 is thinner than the minimum inner diameter of the tip body 11 and has a rear end toward which light from the light source can enter. The optical fiber 12 is inserted from the rear-end opening to the pointed-end opening of the tip body 11, and has an intermediate part that is in contact with the curved part 16, so that the end is vibrated by ultrasonic vibration of the tip body 11. The ultrasound scaler body 2 houses a liquid supply means, an ultrasound-generating means, and a light source, which is attached to the rear end of the ultrasound scaler tip 1. The light source causes light for sterilization in a predetermined wavelength range to enter the rear end of the optical fiber 12.

## Description

### TECHNICAL FIELD

The present invention relates to an ultrasound scaler tip and an ultrasound scaler, and a toothbrush.

### BACKGROUND ART

Conventionally, a ultrasound scaler is known (for example, see Japanese Unexamined Patent Application Publication No. 2012-235979 A), which crushes calculus by transmitting ultrasonic vibration generated by an ultrasonic transducer to the pointed-end part of a probe, supplies an antiseptic solution supplied from a liquid supply means from the pointed-end part of the probe to tooth parts, and then irradiates the supplied antiseptic solution with light directed by a light guide from a light source apparatus, so as to generate hydroxyl radicals for sterilization.

Furthermore, a toothbrush is disclosed (for example, see Japanese Unexamined Patent Application Publication No. H09-140454 A (1997)), which irradiates with light containing ultraviolet rays from a light source from the ends of a brush through the brush made of an optical fiber.

### CITATION LIST

### Patent Literature

Japanese Unexamined Patent Application Publication No. 2012-235979 A
Japanese Unexamined Patent Application Publication No. H09-140454 A (1997)

### SUMMARY OF INVENTION

### Technical Problem

In the case of the ultrasound scaler disclosed in JP Patent Publication (Kokai) No. 2012-235979 A, hydroxyl radicals are generated to perform sterilization, so that a dental scaling part is irradiated with light of the light guide. However, this ultrasound scaler is problematic in that the probe has a thin opening at the pointed end, the area to be irradiated with an even thinner light beam of the light guide is narrow, the area where hydroxyl radicals are generated is limited, and thus the sterilization effect cannot be exhibited sufficiently.

The tooth brush disclosed in JP Patent Publication (Kokai) No. H09-140454 A (1997) is problematic in that ultraviolet rays may adversely affect the interior of the mouth.

The present invention has been achieved noting such problems and an objective of the present invention is to provide an ultrasound scaler tip and an ultrasound scaler having a high sterilization effect. Moreover, another objective of the present invention is to provide a toothbrush having a reduced effect on the interior of the mouth.

### Solution to Problem

To achieve the above objectives, the ultrasound scaler tip according to the present invention is an ultrasound scaler tip, which is used while being attached to an ultrasound scaler body having a light source of light for sterilization in a predetermined wavelength range, and has a tip body and an optical fiber, wherein: the tip body has a tubular shape with a pointed-end opening and a rear-end opening, a rear end that can be attached to the ultrasound scaler body, and an intermediate curved part; and the optical fiber is thinner than the minimum inner diameter of the tip body, has a rear end toward which light can enter from the light source, is inserted from the rear-end opening to the pointed-end opening of the tip body, and has an intermediate part that is in contact with the curved part, so that the pointed end is vibrated by ultrasonic vibration of the tip body.

The ultrasound scaler tip according to the present invention is used with the rear end attached to the ultrasound scaler body. The tip body crushes and removes calculus within the periodontal pocket by ultrasonic vibration. The optical fiber is used for irradiation with light for sterilization within the range where the pointed end is vibrated by the ultrasonic vibration of the tip body. Therefore, the optical fiber enables a wider range of light irradiation compared with a case where the optical fiber is fixed to the tip body, so as to be able to enhance the sterilization effect. The optical fiber has the intermediate part that is in contact with the curved part of the tip body, which ensures the transmission of the ultrasonic vibration of the tip body to the optical fib er.

Light for sterilization in a predetermined wavelength range is preferably light having a wavelength between 400 nm and 420 nm. Light irradiation is preferably performed with the optical fiber in the presence of a hydrogen peroxide solution or hypochlorous acid in order to enhance the sterilization effect. The curved part of the tip body may also have a curved projection located to be in contact with the optical fiber. In this case, the optical fiber comes into contact with or comes out of the projection to increase its vibration width, so that the range of light irradiation, that is, the sterilization range can be more increased.

The tip body of the ultrasound scaler tip according to the present invention preferably has a plane symmetrical shape, a pointed-end part that can be inserted into a periodontal pocket, and a pair of projecting parts that extend to the outer face of the pointed-end part along the longitudinal direction and project vertically with respect to the symmetrical surfaces of the tip body. In this case, calculus can be effectively crushed and removed with the projecting parts.

The pointed end of the optical fiber may project from the pointed-end opening. In this case, the vibration width of the pointed end of the optical fiber is increased, so that the range of light irradiation, that is, the sterilization range can be more increased.

The ultrasound scaler according to the present invention is an ultrasound scaler having the ultrasound scaler tip, which has an ultrasound scaler body, a liquid supply means, an ultrasound-generating means, and a light source, wherein: the ultrasound scaler body houses the liquid supply means, the ultrasound-generating means and the light source, and is attached to the rear-end opening of the ultrasound scaler tip, the liquid supply means is provided so as to be able to supply liquid to the rear-end opening of the tip body and to release the liquid from the pointed-end opening; the ultrasound-generating means is provided to generate ultrasound so as to ultrasonically vibrate the tip body; and the light source is provided so that light for sterilization in a predetermined wavelength range enters the rear end of the optical fiber.

The ultrasound scaler according to the present invention ultrasonically vibrates the tip body by the ultrasound-generating means, and then crushes and removes calculus within the periodontal pocket with the ultrasonic vibration of the tip body. The end of the optical fiber is vibrated by the ultrasonic vibration of the tip body, and then irradiation with light for sterilization is performed from the light source within the vibration range. Therefore, the optical fiber enables a wider range of light irradiation and sterilization compared with a case where the optical fiber is fixed to the tip body. The optical fiber has the intermediate part that comes in contact with the curved part of the tip body, which ensures the transmission of the ultrasonic vibration of the tip body to the optical fiber. Liquid is released by the liquid supply means from the pointed-end opening of the tip body. An antiseptic solution is used as such a liquid, and thus disinfection can be performed together with dental scaling. In particular, light having a wavelength between 400 nm and 420 nm is used as light of the light source, and a hydrogen peroxide solution or hypochlorous acid having a concentration of 3 mass% or less is used as liquid to be supplied from the liquid supply means, so that the sterilization effect can be enhanced safely.

The ultrasound scaler according to the present invention has the ultrasound scaler tip having projecting parts on the outer face of the pointed-end part of the tip body, wherein the ultrasound-generating means preferably has a configuration by which the tip body is ultrasonically vibrated vertically to the longitudinal direction of and vertically to the projecting direction of the projecting parts. In this case, calculus can be effectively crushed and removed using the projecting parts in accordance with the direction of the ultrasonic vibration of the tip body.

The toothbrush according to the present invention has a handle, a head, and a brush, wherein: the head has LED for irradiation with light having a wavelength between 400 nm and 420 nm within the head and provided at an end of the handle; and the brush is made of an optical fiber, and is provided on the head so that the LED light enters and the light that has entered goes out from the end.

The toothbrush according to the present invention is capable of performing irradiation with light having a wavelength between 400 nm and 420 nm from the end of the brush made of an optical fiber when a user holds the handle for toothbrushing. Keeping a hydrogen peroxide solution or hypochlorous acid having a concentration of 3 mass% or less in the mouth followed by toothbrushing makes it possible to generate hydroxyl radicals at locations irradiated with light and to sterilize the locations. Light having a wavelength between 400 nm and 420 nm has a reduced effect on the interior of the mouth and can sterilize the same more safely, compared with ultraviolet rays.

### Advantageous Effects of Invention

According to the present invention, the ultrasound scaler tip and the ultrasound scaler having a high sterilization effect can be provided. Moreover, the toothbrush having a reduced effect on the interior of the mouth can be provided.

### BRIEF DESCRIPTION OF DRAWINGS

Fig. 1 is a side view showing an ultrasound scaler tip according to an embodiment of the present invention.
Fig. 2 is an enlarged plan view showing the pointed-end part of the ultrasound scaler tip shown in Fig. 1.
Fig. 3 is an illustration showing the structure of an ultrasound scaler according to an embodiment of the present invention.
Fig. 4 is an illustration showing the structure of a toothbrush according to an embodiment of the present invention.

### DESCRIPTION OF EMBODIMENTS

Embodiments of the present invention are described below on the basis of drawings.

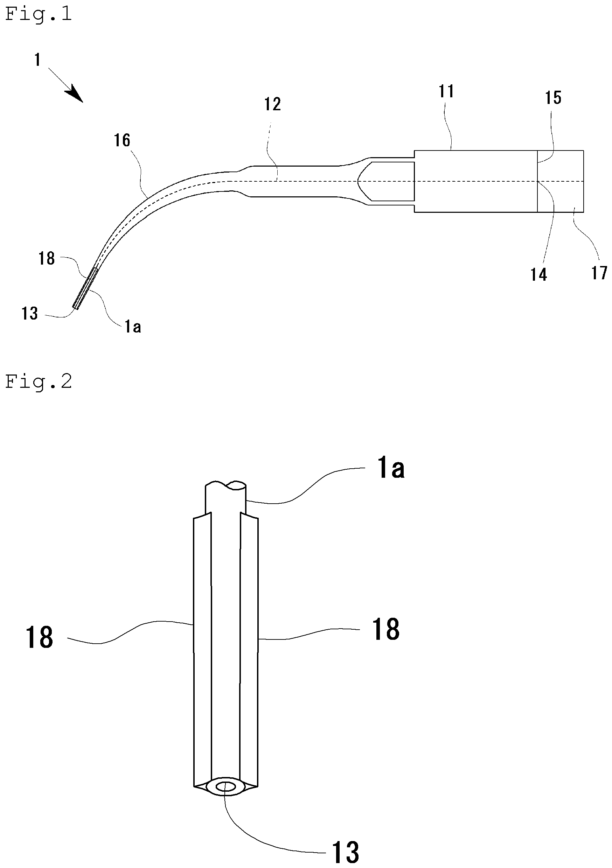

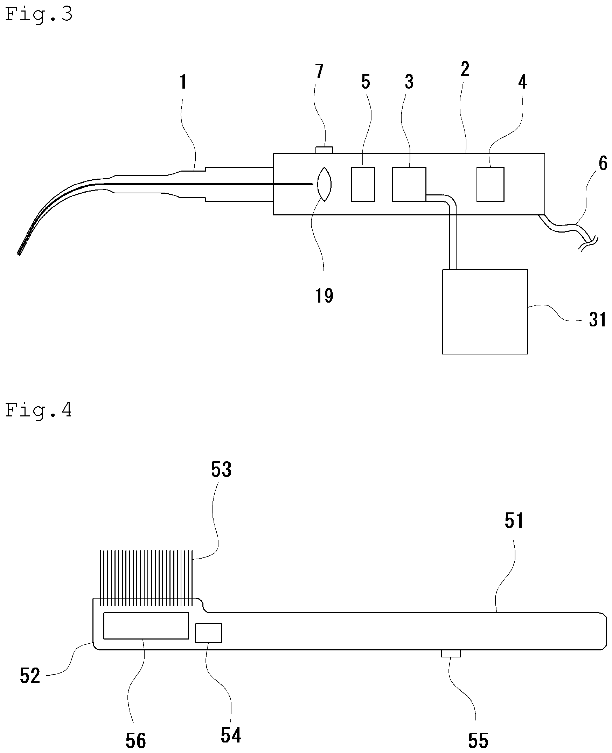

Fig. 1 shows an ultrasound scaler tip according to an embodiment of the present invention and Fig. 2 shows the pointed-end part thereof. Fig. 3 shows an ultrasound scaler having the ultrasound scaler tip.

As shown in Fig. 3, the ultrasound scaler has an ultrasound scaler tip 1, an ultrasound scaler body 2, a liquid supply means 3, an ultrasound-generating means 4, a light source 5, a power supply 6, and a switch 7.

As shown in Fig. 1, the ultrasound scaler tip 1 has a tip body 11 and an optical fiber 12. The tip body 11 has a tubular shape with a pointed-end opening 13 and a rear-end opening 14. The tip body 11 has a rear end 15 that can be attached to the ultrasound scaler body 2 and an intermediate curved part 16. The tip body 11 has the rear end 15 to which a fiber adapter 17 is attached. The tip body 11 has a plane symmetrical shape, and a thin pointed-end part 1a that can be inserted into a periodontal pocket. As shown in Fig. 2, the tip body 11 extends to the outer face of the pointed-end part 1a along the longitudinal direction, and has a pair of fin-shaped projecting parts 18 projecting vertically with respect to the symmetrical surfaces of the tip body 11.

The optical fiber 12 is thinner than the minimum inner diameter of the tip body 11, and has the rear end 15 toward which light can enter from the light source 5. The optical fiber 12 has flexibility, and is inserted from a fiber adapter 17 attached to the rear-end opening 14 to the pointed-end opening 13 of the tip body 11. The optical fiber 12 is disposed leaving a space around and between the fiber and the tip body 11 at the pointed-end opening 13 of the tip body 11. The optical fiber 12 has the intermediate part that is in contact with the curved part 16, so that the end is vibrated as a result of the ultrasonic vibration of the tip body 11.

The ultrasound scaler body 2 houses the liquid supply means 3, the ultrasound-generating means 4 and the light source 5. The ultrasound scaler body 2 has an end attached to the rear end 15 of the ultrasound scaler tip 1. The liquid supply means 3 is provided so as to be able to supply liquid contained within a liquid tank 31 located outside the ultrasound scaler body 2 to the rear-end opening 14 of the tip body 11 and to release the liquid from the pointed-end opening 13. As the liquid, a hydrogen peroxide solution or hypochlorous acid having a concentration of 3 mass% or less is used appropriately.

The ultrasound-generating means 4 is provided to generate ultrasound, so as to ultrasonically vibrate the tip body 11. The ultrasound-generating means 4 has a configuration by which the tip body 11 is ultrasonically vibrated vertically to the longitudinal direction of and vertically (the arrow direction in Fig. 1) to the projecting direction of the projecting parts 18.

The light source 5 is made of LED, and is provided so that light for sterilization in a predetermined wavelength range enters in the form of a laser beam the rear end 15 of the optical fiber 12 via a lens 19. Light for irradiation from the light source 5 is light having a wavelength between 400 nm and 420 nm.

The power supply 6 is composed of a 100V power supply, and is connected to supply electric power to the liquid supply means 3, the ultrasound-generating means 4, and the light source 5 for actuation or light emission. The power supply 6 can be turned on and off via the operation of the switch 7.

Next, the action is explained.

The ultrasound scaler according to the present invention is used for dental treatment, and is specifically used with the pointed-end part 1a of the tip body 11 inserted in a periodontal pocket. When the switch 7 is turned on, the tip body 11 is ultrasonically vibrated by the ultrasound-generating means 4, so as to crush and remove calculus within the periodontal pocket via the ultrasonic vibration of the tip body 11. The ultrasound-generating means 4 ultrasonically vibrates the tip body 11 vertically to the longitudinal direction of and vertically to the projecting direction of the projecting parts 18. In this manner, calculus can be effectively crushed and removed using the projecting parts 18 in accordance with the direction of the ultrasonic vibration of the tip body 11.

The end of the optical fiber 12 is vibrated by the ultrasonic vibration of the tip body 11, irradiation with light for sterilization is performed from the light source 5 within the vibration range. Therefore, the optical fiber 12 enables a wider range of light irradiation compared with a case where the optical fiber is fixed to the tip body 11, so as to be able to enhance the sterilization effect. The optical fiber 12 has the intermediate part that is in contact with the curved part 16 of the tip body 11, which ensures the transmission of the ultrasonic vibration of the tip body 11 to the optical fiber 12. Liquid is released into the periodontal pocket from the pointed-end opening 13 of the tip body 11 by the liquid supply means 3, so that disinfection can be performed together with dental scaling. The light having a wavelength between 400 nm and 420 nm of the light source 5 reacts with the hydrogen peroxide solution or hypochlorous acid having a concentration of 3 mass% or less to generate hydroxyl radicals. In this manner, sterilization can be performed safely for human bodies and the growth of periodontal disease bacteria can be suppressed.

Fig. 4 shows a toothbrush according to an embodiment of the present invention.

As shown in Fig. 4, the toothbrush has a handle 51, a head 52, a brush 53, a power supply 54, and a switch 55. The head 52 has LED 56 therewithin for irradiation with light having a wavelength between 400 nm and 420 nm. The head 52 is provided integrally with an end of the handle 51. The brush 53 is made of an optical fiber, and is provided on the head 52 so as to cause the light of LED 56 to enter and to cause the light that has entered to go out from the end. The brush 53 may be attached by implanting the ends of bristles one by one into the head 52 or by folding the fiber at the center and then implanting the center part into the head 52. The power supply 54 is composed of an electric battery, and is provided within the handle 51. The power supply 54 supplies electric power to the LED 56. The power supply 54 can be turned on and off via the operation of the switch 55.

When a user holds the handle 51 for toothbrushing, the user can perform irradiation with light having a wavelength between 400 nm and 420 nm by turning the switch 55 of the tooth brush on, from the end of brush 53 made of an optical fiber. Keeping a hydrogen peroxide solution or hypochlorous acid having a concentration of 3 mass% or less in the mouth followed by toothbrushing makes it possible to generate hydroxyl radicals at locations irradiated with light and to sterilize the locations. Light having a wavelength between 400 nm and 420 nm has a reduced effect on the interior of the mouth and can sterilize the same more safely, compared with ultraviolet rays.

### REFERENCE SIGNS LIST

- 1: Ultrasound scaler tip
- 1a: Pointed-end part
- 2: Ultrasound scaler body
- 3: Liquid supply means
- 4: Ultrasound-generating means
- 5: Light source
- 6: Power supply
- 7: Switch
- 11: Tip body
- 12: Optical fiber
- 13: Pointed-end opening
- 14: Rear-end opening
- 16: Curved part
- 18: Projecting part
- 51: Handle
- 52: Head
- 53: Brush
- 54: Power supply
- 55: Switch
- 56: LED

## Claims

1. An ultrasound scaler tip, which is used while being attached to an ultrasound scaler body having a light source of light for sterilization in a predetermined wavelength range, and has a tip body and an optical fiber, wherein:
said tip body has a tubular shape with a pointed-end opening, a rear-end opening, a rear end that can be attached to the ultrasound scaler body, and an intermediate curved part; and
said optical fiber is thinner than the minimum inner diameter of said tip body, has a rear end toward which light can enter from said light source, is inserted from said rear-end opening to said pointed-end opening of said tip body, has an intermediate part that is in contact with said curved part so that the pointed-end is vibrated by the ultrasonic vibration of said tip body.

2. The ultrasound scaler tip according to claim 1, wherein said tip body has a plane symmetrical shape, has a pointed-end part that can be inserted into a periodontal pocket, extends to the outer face of said pointed-end part along the longitudinal direction, and has a pair of projecting parts projecting vertically with respect to symmetrical surfaces of the tip body.

3. The ultrasound scaler tip according to claim 1 or 2, wherein the end of said optical fiber projects from said pointed-end opening.

4. An ultrasound scaler, which has the ultrasound scaler tip according to claim 1, 2 or 3, and has
an ultrasound scaler body, a liquid supply means, an ultrasound-generating means, and a light source, wherein:
said ultrasound scaler body houses said liquid supply means, said ultrasound-generating means, and said light source, and is attached to said rear end of said ultrasound scaler tip;
said liquid supply means is provided so that liquid can be supplied to said rear-end opening of said tip body and then released from said pointed-end opening;
said ultrasound-generating means is provided so as to generate ultrasound and ultrasonically vibrate said tip body; and
said light source is provided so that light for sterilization in a predetermined wavelength range enters the rear end of said optical fib er.

5. The ultrasound scaler according to claim 4, which has the ultrasound scaler tip according to claim 2, wherein said ultrasound-generating means has a configuration, by which said tip body is ultrasonically vibrated vertically to the longitudinal direction of and vertically to the projecting direction of said projecting parts.

6. A toothbrush having a handle, a head, and a brush, wherein:
said head has LED within the head for irradiation with light having a wavelength between 400 nm and 420 nm, and is provided at an end of said handle,
said brush is made of an optical fiber, and is provided on said head so that the light of said LED enters and the light that has entered goes out from the end.
